# EUROPÄISCHE PATENTSCHRIFT

(11) **EP 2 730 210 B1**
(45) Veröffentlichungstag und Bekanntmachung des Hinweises auf die Patenterteilung: **28.02.2018**
(21) Anmeldenummer: 13190856.8
(22) Anmeldetag: 30.10.2013
(51) Int. Cl.: A61B 1/00

(54) **Beobachtungsinstrument mit einem hochauflösenden Bildaufnehmer**
Observation instrument with a high-resolution image sensor
Instrument d'observation doté d'un capteur d'images haute résolution

(30) Priorität: 13.11.2012 DE 102012110905
(43) Veröffentlichungstag der Anmeldung: 14.05.2014
(73) Patentinhaber: Karl Storz SE & Co. KG, 78532 Tuttlingen (DE)
(72) Erfinder: Baumann, Harald, 78532 Tuttlingen (DE); Reinacher, Joachim, 78532 Tuttlingen (DE)
(74) Vertreter: Witte, Weller & Partner Patentanwälte mbB

(56) Entgegenhaltungen:
- EP-A2- 2 392 248
- WO-A1-2009/041723
- WO-A2-2006/066022
- DE-A1- 19 750 640
- DE-A1-102005 008 153
- DE-A1-102009 020 262
- JP-A- S61 254 917
- US-A- 5 454 366
- US-A- 5 751 341
- US-A- 5 912 764
- US-B1- 7 280 283

## Beschreibung

Die Erfindung betrifft ein Beobachtungsinstrument mit einem Hohlkörper, der als Hohlschaft eines bestimmten Innendurchmessers ausgebildet ist, wobei in einem Endbereich des Hohlkörpers ein opto-elektronisches Bildaufnehmersystem angeordnet ist, das bildeintrittseitig ein optisches Linsensystem mit einem zylinderförmigen Abschnitt und anschließend einen Bildsensor aufweist, der das vom optischen Linsensystem kommende Bild in elektrische Bildsignale wandelt, wobei sich die optische Achse des Linsensystems in Richtung der Längsachse des Hohlkörpers erstreckt, wobei der Außendurchmesser des zylinderförmigen Abschnitts des Linsensystems geringer ist als der Innendurchmesser des Hohlkörpers, und zwar derart, dass zwischen einer Innenseite des Hohlkörpers und der Außenseite des optischen Linsensystems ein ausreichender Zwischenraum verbleibt, durch den weitere Bauelemente im Hohlkörper durchführbar sind, wobei bei einer Blickrichtung von größer 0°bis kleiner 90°aus der optischen Achse d es Bildsystems heraus bildeintrittseitig am Linsensystem ein distales Umlenkprisma angeordnet ist, wobei das distale Umlenkprisma einen sich seitlich über den Außendurchmesser des zylinderförmigen Abschnitts des Linsensystems hinauserstreckenden Abschnitt aufweist, wobei die Bildeintrittsebene des Bildsensors parallel zur optischen Achse verläuft, und wobei sich das distale Umlenkprisma seitlich in derselben Richtung über den Außendurchmesser des zylinderförmigen Abschnitts des Linsensystems hinauserstreckt, in der die Bildeintrittsebene des Bildsensors zum zylinderförmigen Abschnitt des Linsensystems seitlich nach außen versetzt ist.

Derartige Beobachtungsinstrumente mit einem Hohlkörper in Form eines Schaftes finden insbesondere als Endoskope oder als Exoskope Einsatz. Aus der DE 10 2010 007 394 A1 ist ein medizinisches Beobachtungsinstrument bekannt, in dessen Schaft im distalen Endbereich ein opto-elektronisches Bildaufnehmersystem angeordnet ist.

Bei zahlreichen Beobachtungsinstrumenten verläuft die Blickrichtung geradeaus, das heißt diese verläuft in der Längsachse des Schaftes. In manchen Einsatzbereichen, insbesondere im medizinischen Bereich, ist es aber erwünscht, dass solche Beobachtungsinstrumente eine Blickrichtung aufweisen, die aus der geradlinigen Blickrichtung, der sogenannten 0°-Blickrichtung, abweich en, wobei die Blickrichtung zwischen 0° und bis zu 90°aus der geradlinigen Blickrichtun g abweicht.

Dadurch können Hohlräume, wie beispielsweise der Innenraum einer Blase, weiträumig eingesehen werden, insbesondere dann, wenn der sich geradlinig und längs erstreckende Schaft durch einen sich lang erstreckenden Kanal, beispielsweise eine Harnröhre, in diesen zu beobachtenden Hohlraum hineingeführt werden muss.

Die optische Achse des eigentlichen Linsensystemes, das das eintretende Bild zu dem optischen Sensor führt, erstreckt sich in Richtung der Längsachse des Schaftes. Dadurch ist es möglich, die Linsensysteme durch übliche Linsen mit kreisförmigem Querschnitt herzustellen.

Um das aus der geneigten Blickrichtung eintreffende Licht bzw. Bild nun diesem sich in der optischen Achse erstreckenden Linsensystem zuführen zu können, ist bildeintrittseitig am Linsensystem ein distales Umlenkprisma angeordnet. Dieses Umlenkprisma hat die Aufgabe, die unter dem jeweiligen Blickwinkel zwischen 0° und 90° eintreffenden Licht- bzw. Bildstrahlen dem optischen Linsensystem möglichst in Richtung der optischen Achse zuzuführen.

Bei diesen Beobachtungsinstrumenten wird das Bild von dem optischen Linsensystem auf einen Bildsensor projiziert, der die optischen Bildinformationen in elektrische Bildsignale wandelt. Diese elektrischen Bildsignale werden dann über entsprechende Leitungen einem Bildwandlungssystem oder einem Monitor zugeführt.

Die Technologie der Bildsensoren hat in den letzten Jahren eine erhebliche Entwicklung durchgemacht, insbesondere was die Auflösung betrifft. So sind gegenwärtig hochauflösende Bildsensoren bereits auf dem Markt, deren Pixelgrößen kleiner als 3 µm sind.

Die Hohlschäfte von Beobachtungsinstrumenten weisen einen Durchmesser auf, der im Wesentlichen dadurch bestimmt wird, wie groß die Öffnung ist, durch die der Schaft des Beobachtungsinstrumentes hindurchgeführt werden kann.

Insbesondere im medizinischen Bereich sind diese Öffnungen durch bestimmte Organe vorgegeben, beispielsweise durch den Durchmesser einer Harnröhre oder auch die Anatomie des Menschen, in dem beobachtet werden soll.

So sind beispielsweise bei Kindern wesentlich durchmessergeringere Schäfte einsetzbar als bei Erwachsenen. Im HNO-Bereich und auch in der Hirnchirurgie stehen oftmals nur sehr durchmessergeringe Öffnungen oder Kanäle zum Einführen von solchen Hohlschäften von Beobachtungsinstrumenten zur Verfügung.

In zahlreichen Fällen ist das Beobachtungsinstrument nicht nur ein reines Beobachtungsinstrument, sondern durch den Schaft sollen auch noch medizinische Eingriffe vorgenommen werden können. Daher kann das opto-elektronische Bildaufnehmersystem nicht das gesamte innere Lumen des Hohlkörpers belegen, sondern nur Teile davon, so dass neben dem opto-elektronischen Bildaufnehmersystem noch ein ausreichend großer Zwischenraum verbleibt, um weitere Bauelemente hindurchzuführen.

Da durch diesen Zwischenraum beispielsweise Instrumente hindurchgeführt werden, die aus dem Körperinneren nach dem Eingriff kontaminiertes Gewebe durch den Schaft abführen, muss sichergestellt sein, dass eine hermetische Abriegelung zwischen dem opto-elektronischen Bildaufnehmersystem und diesem Zwischenraum erfolgt, damit keine Kontaminationen oder Verschmutzungen in dieses System eindringen können. Ferner muss dieses System auch deswegen hermetisch von dem Zwischenraum abgetrennt werden, da dieser nach einen Eingriff gespült, gereinigt und desinfiziert werden muss. Die dabei eingesetzten chemisch aggressiven Mittel könnten das opto-elektronische System beschädigen.

Daher wird bei dieser Technologie mit der sogenannten Mehrrohrtechnik gearbeitet. Das bedeutet, das opto-elektronische Bildaufnehmersystem wird in einen Innenschaft integriert, der in den eigentlichen Hohlkörper, der dann als Außenschaft bezeichnet wird, eingebracht wird. Dabei sind Anordnungen üblich, bei denen der Innenschaft entweder koaxial in dem Außenschaft angeordnet ist, so dass um dessen Außenseite ein ringförmiger Zwischenraum verbleibt, durch den dann weitere Bauelemente oder Instrumente oder auch andere Schäfte hindurchgeführt werden können. Oder, der Innenschaft wird längs einer Mantellinie an der Innenseite des Außenschaftes angeordnet, so dass dann ein mondsichelartiger Zwischenraum verbleibt. Demzufolge entspricht üblicherweise der Außendurchmesser des zylinderförmigen Abschnittes des Linsensystemes dem Innendurchmesser eines Innenschaftes, der in den eigentlichen Hohlkörper eingesetzt ist.

Bei den Beobachtungssystemen mit von der geradlinigen 0°-Blickrichtung abweichenden Blickrichtungen ist das distale Umlenkprisma notwendig. Da dieses ebenfalls in dem vom Zwischenraum hermetisch abgeschlossenen opto-elektronischen Bildaufnehmersystem angeordnet ist, ist dessen radiale Erstreckung ebenfalls auf den Durchmesser des zylinderförmigen Abschnittes beschränkt, da es in diesem Innenschaft oder einer entsprechenden Hülse, die diesen Abschnitt des optischen Systemes umgibt, aufgenommen ist.

Es wurde nun festgestellt, dass durch die Weiterentwicklung der hochauflösenden Bildsensoren durch ein solches opto-elektronisches Bildaufnehmersystem weniger Bildinformation auf den Bildsensor geleitet wird, als dieser eigentlich leisten könnte. Die hohe Auflösung wird durch die sogenannte numerische Apertur bestimmt, also den Winkel, den ein Bildstrahlbündel einschließt, das auf einen Pixelpunkt des Bildsensors auftrifft. Je höher die numerische Apertur ist, also je größer dieser Winkel ist, umso schärfer und hochaufgelöster ist der Bildpunkt.

Es wurde nun im praktischen Einsatz festgestellt, dass mit den vorgegebenen Dimensionen, sprich dem Durchmesser des optischen Linsensystemes, nicht so viel Bildinformation auf einen hochauflösenden Bildsensor geleitet werden kann, wie dieser erfassen kann.

Eine Lösung des Problemes wäre, den Durchmesser des optischen Linsensystemes bei gleichbleibender Bildaufnahmefläche und Auflösung eines Bildsensors zu vergrößern. Dies geht aber deswegen nicht, da sonst der Zwischenraum zwischen dem opto-elektronischen Bildaufnehmersystem und der Innenseite des Hohlkörpers gering würde, so dass man keine Instrumente mehr hindurchführen könnte bzw. nicht genügend optische Fasern für die Beleuchtung nach distal führen könnte.

Wie bereits erwähnt ist die Größe bzw. der Durchmesser des Außenschaftes dadurch bestimmt, in welche Öffnung ein solcher Schaft eines Beobachtungsinstrumentes eingeschoben werden kann. Im Bereich der technischen Endoskopie wären das beispielsweise vorhandene Bohrungen, zum Beispiel um den Innenraum eines Brennraumes oder dergleichen zu untersuchen, die aus irgendwelchen Gründen nicht vergrößert werden können.

WO 2006/066022 A2 offenbart ein Beobachtungsinstrument mit einem Hohlkörper, wobei in einem Endbereich des Hohlkörpers ein Bildaufnehmersystem mit mehreren abgestuften Durchmesserabschnitten angeordnet ist, das bildeintrittsseitig ein optisches System und anschließend einen Bildsensor aufweist, der das vom optischen Linsensystem kommende Bild in elektrische Bildsignale wandelt. Ferner weist das Beobachtungsinstrument ein proximales Umlenkprisma auf, das zwischen dem optischen System und dem Bildsensor angeordnet ist, wobei eine Bildaustrittsfläche des proximalen Umlenkprismas auf der Bildeintrittsebene des Bildsensors liegt. Eine optische Achse des optischen Systems verläuft in Richtung einer Längsachse des Hohlkörpers, wobei zwischen einer Innenseite des Hohlkörpers und der Außenseite des optischen Systems ein ausreichender Zwischenraum verbleibt, durch den mehrere Lichtleiter durchführbar sind, wobei bei einer in Bezug auf die optische Achse schrägen Blickrichtung bildeintrittsseitig am optischen System ein distales Umlenkprisma angeordnet ist.

Aus der US 5,751,341 A2 ist ein Stereo-Endoskop bekannt, das einen Schaft und ein Gehäuse aufweist, wobei sich im Schaft und im Gehäuse ein opto-elektronisches Bildaufnehmersystem erstreckt, das ein distales Umlenkprisma und ein proximales Umlenkprisma aufweist, wobei das proximale Umlenkprisma im Gehäuse platziert und von einem Bildaufnehmer beabstandet angeordnet ist. Zwischen dem distalen Umlenkprisma und dem proximalen Umlenkprisma erstreckt sich ein Linsensystem. Der Schaft weist ein Außenrohr auf. Das Linsensystem ist ein einem Innenrohr aufgenommen. Zwischen dem Außenrohr und dem Innenrohr sind Lichtfasern angeordnet. Der Bildaufnehmer und das distale Umlenkprisma sind zumindest abschnittweise außerhalb einer zyindrischen Gestalt des Innenrohres angeordnet.

Es ist daher Aufgabe der vorliegenden Erfindung, hier Abhilfe zu schaffen und ein Beobachtungsinstrument der eingangs genannten Art mit einer Blickrichtung, die von der geradlinigen Blickrichtung abweicht, dahingehend zu verbessern, dass so viel Bildinformation eingeführt werden kann, dass auch die hochauflösenden Bildsensoren in ihren Fähigkeiten genutzt werden können, insbesondere Bildsensoren mit Pixelgrößen kleiner 3 µm.

Erfindungsgemäß wird die Aufgabe dadurch gelöst, dass zwischen dem optischen Linsensystem und dem Bildsensor ein proximales Umlenkprisma angeordnet ist, dessen Bildaustrittsfläche auf der Bildeintrittsebene des Bildsensors liegt, und dass das den Bildsensor aufweisende opto-elektronische Bildaufnehmersystem in einem distalen Endbereich des als Hohlschaft ausgebildeten Hohlkörpers aufgenommen ist.

Diese Maßnahmen haben verschiedene Vorteile. Der zylindrische Abschnitt des optischen Linsensystemes kann eine solche Größe aufweisen oder beibehalten, die ausreichend ist, um in dem Hohlkörper noch einen ausreichenden Zwischenraum, sei es ein ringförmiger oder ein mondsichelförmiger, aufrecht zu erhalten.

Dadurch, dass das diesem optischen Linsensystem bildeintrittseitig vorgeschaltete distale Umlenkprisma sich seitlich über den Außendurchmesser des zylinderförmigen Abschnittes hinaus erstreckt, kann mehr Bildinformation über das Umlenkprisma in das optische Linsensystem eingekoppelt werden, als durch ein distales Umlenkprisma, das sich nur innerhalb des Durchmessers des optischen Systemes erstreckt, da der abbildungserzeugende Strahlengang im Prisma teilweise außerhalb des zylinderförmigen Abschnittes des optischen Linsensystems verläuft. Dadurch kann durch das optische Linsensystem mehr Bildinformation zu dem hochauflösenden Bildsensor geleitet werden. Dabei muss weder der Durchmesser des Hohlkörpers vergrößert werden, noch wird der Zwischenraum neben dem opto-elektronischen Bildaufnehmersystem wesentlich beeinträchtigt, mit Ausnahme am distalen Ende, an dem sich das Umlenkprisma seitlich über die Außenkontur des zylindrischen Abschnittes hinaus erstreckt.

Diese seitliche Hinauserstreckung belegt aber nicht den ganzen Zwischenraum, denn diese erfolgt nur in einer seitlichen Richtung. Dadurch bleibt nach wie vor ein ausreichender Zwischenraum zur Verfügung, um beispielsweise Lichtleiter oder dergleichen von einem proximalen zu einem distalen Ende in dem Zwischenraum im Hohlkörper zu führen. Die Lichtleiter können zum Beispiel an der der seitlichen Hinauserstreckung radial gegenüberliegenden Seite am Bildaufnehmersystem vorbei geführt werden. Es wurde festgestellt, dass durch eine entsprechende längere Ausbildung des distalen Umlenkprismas im Sinne einer seitlichen Erstreckung über die Querschnittsfläche des optischen Systemes hinaus es ermöglicht wird, dem optischen Linsensystem so viel Bildinformation zuzuführen, dass auch hochauflösende Bildsensoren optimal genutzt werden können.

Das Resultat ist, dass bei gleichbleibendem Schaftdurchmesser und letztendlich unverändertem Durchmesser des zylinderförmigen Abschnittes eines optischen Linsensystemes durch diese Ausgestaltung des distalen Umlenkprismas wesentlich schärfere oder hochauflösendere Bilder möglich sind. Durch Nutzen des sich seitlich über den Außendurchmesser des Linsensystems hinaus erstreckenden Abschnitts des distalen Umlenkprismas für den Strahlengang ist bei einer Schrägblickoptik ein großer Blickwinkel mit einer hohen numerischen Apertur möglich. Dadurch kann das Beobachtungsergebnis wesentlich verbessert werden. Das ist bei Beobachtungsinstrumenten mit von der 0°-Blickrichtung abweichenden Blickrichtung deswegen von großer Bedeutung, da mit solchen Instrumenten ein Rundumblick in einem Hohlorgan, beispielsweise einer Blase, erzielt werden kann, was nunmehr mit einer hochauflösenden Bildqualität erreicht werden kann.

Die Bildeintrittsebene des Bildsensors verläuft parallel zur optischen Achse. Es wird ein proximales oder zweites Umlenkprisma zwischengeschaltet, um die vom optischen Linsensystem bildaustrittsseitige Bildinformation entsprechend auf die Bildeintrittsebene des Bildsensors umzulenken.

In einer Ausgestaltung der Erfindung ist die Bildeintrittsebene des Bildsensors gegenüber dem zylinderförmigen Abschnitt des Linsensystems radial nach außen versetzt.

Da entsprechend der Erfindung das distale Umlenkprisma sich seitlich in einen Bereich hinauserstreckt und dieser Bereich sich dann für eine geradlinige Hindurchführung eines Instrumentenkanales nicht mehr eignet, kann der seitliche Bereich proximal hinter dem optischen Linsensystem liegend zur Aufnahme des Bildsensors herangezogen werden. Je nachdem, wie viel Platz zur Verfügung steht, kann dann dessen Bildeintrittsebene mehr oder weniger seitlich bzw. radial nach außen versetzt zum zylinderförmigen Abschnitt des Linsensystemes liegen. Dabei kann der Bildsensor auch noch leicht gegenüber der optischen Achse angestellt sein. Vorteilhafterweise reicht daher das distale Umlenkprisma in derselben Richtung radial nach außen, in der der Bildsensor radial nach außen versetzt ist.

Es ist auch von Vorteil, dass zwischen dem optischen Linsensystem und dem Bildsensor ein proximales Umlenkprisma angeordnet ist, dessen Bildaustrittsebene auf der Bildeintrittsebene des Bildsensors liegt.

In einer weiteren Ausgestaltung der Erfindung weist das distale Umlenkprisma einen Öffnungswinkel von größer/gleich 50° auf.

Ein solch großer Öffnungswinkel erlaubt es, einen großen Bereich einzusehen.

In einer weiteren Ausgestaltung der Erfindung ist das distale Umlenkprisma derart aufgebaut, dass innerhalb des Umlenkprismas eine Strahlaufweitung von kleiner/gleich 30 % erfolgt.

Diese Maßnahme hat den Vorteil, dass man kein überdimensioniertes distales Umlenkprisma braucht, dieses also in axialer Richtung gesehen relativ kurz baut.

In einer weiteren Ausgestaltung der Erfindung ist vor dem distalen Umlenkprisma eine negativ brechende Linse angeordnet, die den eintretenden Strahlen einen paralleleren Verlauf verleiht.

Diese Maßnahme hat den Vorteil, dass durch die Parallelität die Strahlen das optisch Linsensystem so durchlaufen, dass wenig Abschattungen oder an den Seitenflächen reflektiertes Licht im System erzeugt wird, das als Falschlicht auf den Sensor fallen könnte und dadurch die Bildauflösung verschlechtern könnte. Auch diese Maßnahme erlaubt eine relativ kompakte Bauweise des distalen Umlenkprismas.

In einer weiteren Ausgestaltung der Erfindung ist der Innenraum des Hohlkörpers, zumindest im Endbereich des Hohlkörpers, in dem das opto-elektronische Bildaufnehmersystem angeordnet ist, durch zumindest einen Trennsteg derart aufgeteilt, dass in einem dadurch entstehenden Raum das opto-elektronische Bildaufnehmersystem passend aufnehmbar ist.

Diese Maßnahme hat den Vorteil, dass nunmehr die Möglichkeit geschaffen wird, durch Vorsehen des Trennsteges einen separaten Raum im Hohlkörper vorzusehen, in den das opto-elektronische Bildaufnehmersystem aufgenommen werden kann.

Durch die Trennwand kann somit eine hermetische Abtrennung gegenüber dem Zwischenraum geschaffen werden, in den die weiteren Bauelemente durch den Hohlkörper hindurchgeführt bzw. eingebaut (z.B. Lichtleiter) werden können.

Der Begriff "passend" bedeutet, dass der Trennsteg den Innenraum des Hohlkörpers so auftrennt, dass in dem einen dadurch entstehenden Raum das gesamte opto-elektronische Bildaufnehmersystem, also auch mit dem seitlich überstehenden distalen Umlenkprisma, passend aufgenommen werden kann. Auf der anderen Seite des Trennsteges ist dann der Zwischenraum vorhanden, durch den die weiteren Bauelemente durch den Hohlkörper hindurchgeführt oder weitere Bauelemente, wie beispielsweise Lichtleiter, aufgenommen werden können. Im einfachsten Fall ist nur ein Trennsteg vorhanden, der sich in Richtung der Längserstreckung des Hohlkörpers erstreckt, so dass dieser Trennsteg beispielsweise unmittelbar bei der originären Herstellung des Hohlkörpers mit hergestellt werden kann und einstückig mit dem Hohlkörper ausgebildet ist. Es können auch mehrere Trennstege vorgesehen sein, ein Raum muss auf jeden Fall so ausgebildet sein, dass darin passend das opto-elektronische Bildaufnehmersystem aufgenommen werden kann.

In einer weiteren Ausgestaltung der Erfindung ist der Trennsteg der Außenkontur des seitlich überstehenden Abschnittes des distalen Umlenkprismas angepasst.

Wie bereits erwähnt steht dieser seitlich überstehende Abschnitt des distalen Umlenkprismas seitlich vom zylinderförmigen Abschnitt des optischen Systemes vor, aber eben nur auf einer Seite. Entspricht, im Querschnitt gesehen, die Breite dieses Abschnittes dem Durchmesser des zylindrischen Abschnitts des optischen Systems, so stehen links und rechts von diesem radial seitlich überstehenden Abschnitt noch Räume zur Verfügung. Wird nun der Trennsteg, im Querschnitt gesehen, dieser Außenkontur des überstehenden Abschnitt des distalen Umlenkprismas angepasst, stehen über große Längenabschnitte, ggf. über den gesamten Längenabschnitt, diese Räume für andere Bauelemente zur Verfügung.

In einer weiteren Ausgestaltung der Erfindung ist der zumindest eine Trennsteg derart ausgebildet, dass das opto-elektronische Bildaufnehmersystem zur Montage von distal nach proximal in einen durch diesen Trennsteg entstehenden Raum einführbar ist.

Diese Maßnahme hat den Vorteil, dass das gesamte opto-elektronische System als eine kompakte Einheit vorgefertigt werden kann und dann von distal in den offenen Schaft bzw. in den offenen Raum, der durch den Trennsteg gebildet wird, eingeschoben werden kann.

In einer weiteren Ausgestaltung der Erfindung erstreckt sich der radial seitlich vorstehende Abschnitt des distalen Umlenkprismas bis an den Trennsteg heran.

Diese Maßnahme hat den Vorteil, dass, je nachdem von welcher Blickrichtung man ausgeht, eine optimale Abstimmung zwischen der seitlichen Erstreckung des Abschnittes des distalen Umlenkprismas und der Geometrie und Lage des Trennsteges erzielt werden kann. Es wird dabei zumindest ein so großer Luftspalt freigelassen, dass, wie zuvor erwähnt beispielsweise beim Montieren diese Baueinheit von distal in diesen Raum eingeschoben werden kann.

In einer weiteren Ausgestaltung der Erfindung erstreckt sich der radial seitlich vorstehende Abschnitt des distalen Umlenkprismas zumindest bis zu einer Ebene, die durch die Bildeintrittsebene des Bildsensors aufgespannt ist. Oder umgekehrt ausgedrückt liegt diese Bildeintrittsebene des Bildsensors auf Höhe des äußeren Endes des seitlich vorstehenden Abschnittes.

Diese Geometrie erlaubt eine günstige Anordnung des Bildsensors und eine optimale Ausnützung dessen Auflösung.

In einer weiteren Ausgestaltung der Erfindung erstreckt sich der radial seitlich vorstehende Abschnitt des distalen Umlenkprismas über eine Ebene hinaus, die durch die Rückseite des Bildsensors aufgespannt ist.

Der Bildsensor ist üblicherweise ein plattenförmiger Chip, der in einem Hohlkörper aufgrund seiner Flächengeometrie parallel zur Längsachse des Hohlkörpers liegend angeordnet werden kann. Der seitliche Bereich, der durch den seitlich vorstehenden Abschnitt des distalen Umlenkprismas ohnehin wenig nutzbar ist, kann durch den Bildsensor in einem weiteren proximalen Abschnitt belegt werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschreiben und erläutert. Es zeigen:
- Figur 1: einen Längsschnitt eines distalen Endbereiches eines erfindungsgemäßen Beobachtungsinstrumentes entlang einer Linie I-I wie sie in Figur 3 dargestellt ist;
- Figur 2: einen Querschnitt längs der Linie II-II in Figur 1;
- Figur 3: einen Querschnitt längs der Linie III-III in Figur 1;
- Figur 4: einen Querschnitt längs der Linie IV-IV in Figur 1; und
- Figur 5: das distale Umlenkprisma sowie den Strahlenverlauf darin.

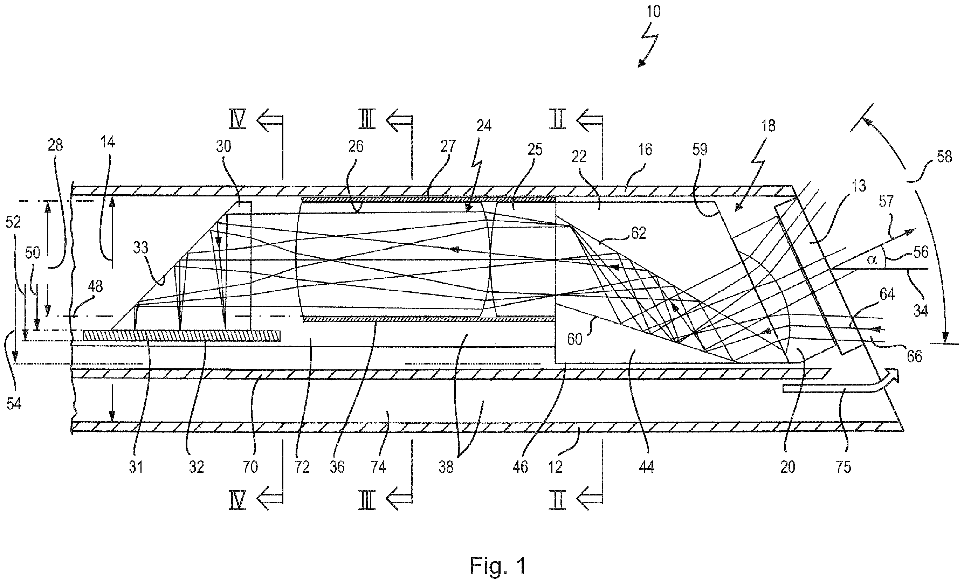

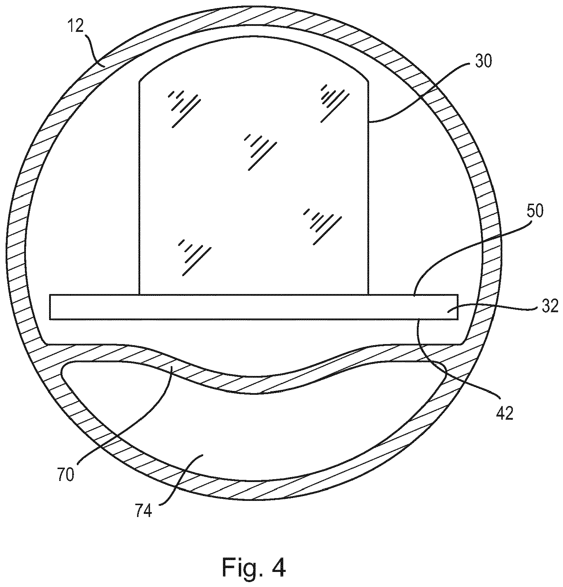

Ein in den Fig. 1 bis 5 dargestelltes Ausführungsbeispiel eines Beobachtungsinstrumentes ist in seiner Gesamtheit mit der Bezugsziffer 10 bezeichnet.

Das Beobachtungsinstrument 10 weist einen geradlinig verlaufenden steifen Hohlkörper in Form eines Hohlschaftes 12 eines bestimmten Innendurchmessers 14 auf. Der Hohlkörper kann nur ein kurzer Stummel oder ein langerstreckter Schaft sein. Der Schaft kann starr oder flexibel sein. In einem distalen Endbereich 16 des Hohlschafts 12 ist ein opto-elektronisches Bildaufnehmersystem 18 aufgenommen. Distalseitig ist der Hohlschaft über ein Deckglas 13 abgeschlossen, das über hier nicht dargestellte Kittmaterialien einen dichtenden Abschluss des distalen Endes des Hohlschafts 12 bildet. Das Deckglas 13 ist transparent und erlaubt somit den Eintritt eines Beobachtungslichtes, sprich eines Bildes, das durch das Beobachtungsinstrument 10 erfasst werden soll.

Das opto-elektronische Bildaufnehmersystem 18 weist eine Negativlinse 20 auf, die einerseits an der inneren Seite des Deckglases 13 angrenzt. Andererseits ruht die Negativlinse 20 auf einem distalen ersten Umlenkprisma 22.

Proximalseitig ist das Umlenkprisma 22 durch ein übliches optisches Linsensystem 24 weitergeführt, das sich aus einer Vielzahl an Linsen 25 zusammensetzt.

Dieses optische Linsensystem 24 stellt einen zylindrischen Abschnitt 26 dar, der in einer diese umgebenden Hülse 27 aufgenommen ist.

Der Außendurchmesser 28 des zylindrischen Abschnittes 26 ist wesentlich geringer, etwa die Hälfte, als der Innendurchmesser 14 des Hohlschaftes 12.

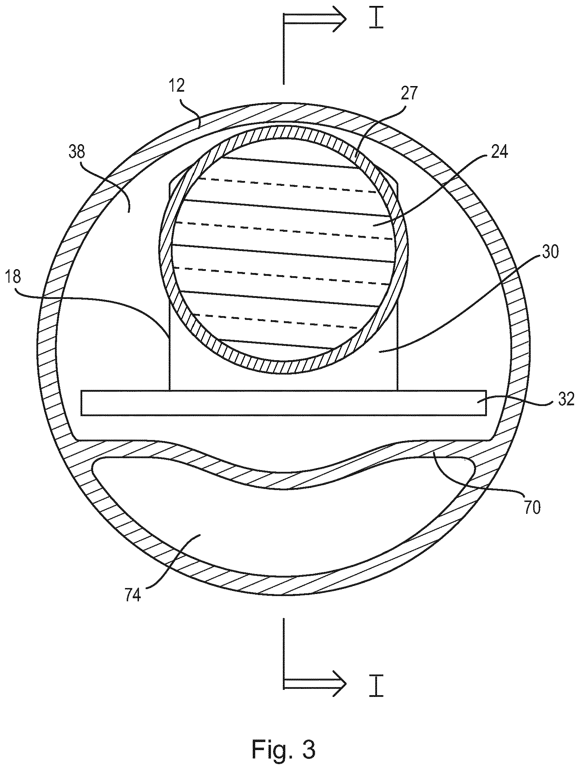

Das optische Linsensystem 24 bzw. die dessen zylindrischen Abschnitt 26 umgebende Hülse 27 ist so im Hohlschaft 12 angeordnet, wie das insbesondere aus dem Querschnitt von Fig. 3 ersichtlich ist, dass eine äußere Mantellinie der Hülse 27 an einer inneren Mantellinie der Innenseite des Hohlschaftes 12 zum Liegen kommt.

Dadurch entsteht ein etwa mondsichelförmiger Zwischenraum 38 um diesen zylindrischen Abschnitt 26 des optischen Linsensystemes 24 herum.

Die optische Achse 34, wie das insbesondere aus Fig. 1 ersichtlich ist, erstreckt sich in Richtung der Längsachse des Hohlschaftes 12, jedoch entsprechend seitlich versetzt zu dieser.

Proximalseitig schließt sich an das optische Linsensystem 24 ein proximales zweites Umlenkprisma 30 an, das dazu vorgesehen ist, um das von dem optischen Linsensystem 24 kommende Bild um 90° auf einen Bildsensor 32 umzulenken. Dabei liegt die Bildaustrittsfläche 31 des proximalen Umlenkprismas 30 direkt auf der Bildeintrittsebene 50 des Bildsensors. Das proximale Umlenkprisma 30 weist eine Umlenkfläche 33 auf, die in einem Winkel von 45° zur optischen Achse 34 steht, um somit eine 90°-Umlenkung von Lichtstrahlen zu bewirken, die das optische Linsensystem 24 proximalseitig verlassen.

Das distale Ende des Beobachtungsinstrumentes 10 ist so ausgebildet, dass es einen Schrägblickwinkel 56 von 25° gegenüber der optischen Achse 34 erlaubt. Das Deckglas 13 und die Negativlinse 20 stehen ebenfalls unter diesem Schrägblickwinkel α, so dass Licht oder Bildinformation aus diesem Blickwinkel ins Bildaufnehmersystem 18 eintreten bzw. von diesem aufgenommen werden kann.

Der Öffnungswinkel 58 des Bildaufnehmersystemes beträgt 50° oder mehr.

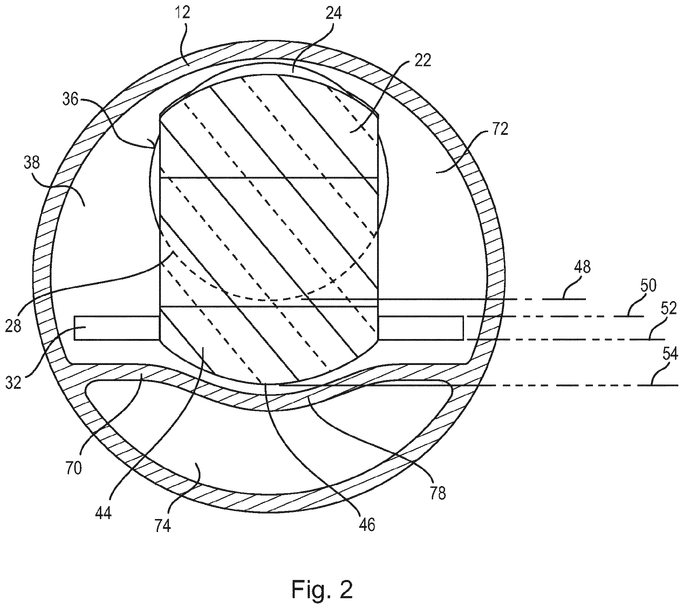

Insbesondere aus den Schnittdarstellungen von Fig. 1 und 2 ist zu erkennen, dass das distale Umlenkprisma 22 einen Abschnitt 44 aufweist, der sich radial über die Außenkontur des zylindrischen Abschnittes 26 des optischen Linsensystemes 24 hinaus erstreckt.

Ferner ist aus Fig. 1 zu erkennen, dass sich dieser Abschnitt 44 auch über die Bildeintrittsebene 50 des Bildsensors 32 hinaus erstreckt. Ferner ist zu erkennen, dass sich dieser Abschnitt 44 auch noch über die Rückseite 42 des Bildsensors 32 hinaus erstreckt, also auch über die von dieser Rückseite aufgespannte Ebene 52.

Ferner ist der Fig. 2 zu entnehmen, dass sich dieser Abschnitt 44 sehr nahe an einen Trennsteg 70 heran erstreckt, der den Innenraum des Hohlschafts 12 in einen ersten Raum 72, in dem das opto-elektronische Bildaufnehmersystem 18 aufgenommen ist, und einen zweiten Raum 74 aufteilt. Es ist nunmehr möglich den überstehenden Abschnitt 44 zusätzlich für den Strahlengang zu nutzen. Dies eröffnet bei solchen Schrägblickoptiken einen großen Blickwinkel mit einer hohen numerischen Apertur.

Insbesondere aus der Schnittdarstellung von Fig. 2 ist zu erkennen, dass der seitlich vorstehende Abschnitt 44 des distalen Umlenkprismas 22 an seinem äußeren Ende abgerundet ist.

Daher ist auch in diesem Bereich der Trennsteg 70 mit einer entsprechenden Krümmung 78 versehen.

Die Krümmung 78 ist dabei dieser Kontur der äußeren Kante 46 des seitlich vorstehenden Abschnittes 44 des distalen Umlenkprismas 22 angepasst.

Wie aus Fig. 1 und 2 zu entnehmen, erstreckt sich somit diese Kante 46 bis an eine Ebene 54 heran, die durch die maximale seitliche Ausdehnung des Abschnittes 44 aufgespannt wird.

In Fig. 1 ist durch einen Pfeil 75 angedeutet, dass in dem Raum 74, der durch die Trennwand 70 geschaffen wird, weitere Bauelemente, beispielsweise Lichtleiter, angeordnet werden können, um Beleuchtungslicht dem distalen Ende des Beobachtungsinstrumentes 10 zuzuführen. Aus Fig. 1 ist auch zu erkennen, dass die Trennwand 70 etwas vor dem abgeschrägten Ende des Hohlschaftes 12 endet, so dass diese Lichtleiter, wie man der Krümmung des Pfeiles 75 entnehmen kann, hier entsprechend gekrümmt, sprich parallel zur Blickrichtung 57, geführt werden können. Die distalen Enden der hier nicht dargestellten Lichtleiter werden dann wie üblich in entsprechende Kitte eingebettet und bilden mit dem Deckglas 13 zusammen einen dichten Abschluss zumindest des Raumes 72. Es können auch am distalen Endbereich LED's angeordnet werden, die das Beleuchtungslicht erzeugen und abstrahlen. Deren Versorgungsleitungen werden im Raum 74 nach proximal geführt. Das Deckglas 13 bildet mit dem Trennsteg 70 einen distalen Abschluss des Raumes für das opto-elektronische Bildaufnahmesystem 18.

Insbesondere aus den Schnittdarstellungen von Fig. 2, 3 und 4 ist ersichtlich, dass "neben" dem Bildaufnehmersystem zumindest im Bereich des optischen Linsensystemes 24 und den Umlenkprismen 22 und 30 noch ausreichend Raum, zum Beispiel der Zwischenraum 38, zur Verfügung steht, um beispielsweise dort weitere Bauteile, beispielsweise Lichtleiter, aufzunehmen.

Die Funktionsweise des Beobachtungsinstrumentes ist nun wie folgt.

In Fig. 1 sind drei Strahlenbündel dargestellt, die zwei seitlich äußere Strahlenbündel sowie ein mittig zentrales Strahlenbündel repräsentieren. Von dem in der Fig. 1 dargestellten unteren Strahlenbündel 66 ist der Mittenstrahl 64 mit einem Pfeil versehen, so dass anhand dieses Mittelstrahls 64 der Strahlengang verfolgt werden kann.

Das einfallende Licht bzw. die Bildinformation treten durch das Deckglas 13 in die Negativlinse 20 ein. Diese Frontlinse ist aufgrund des großen Öffnungswinkels eine Negativlinse. Die Negativlinse weitet die einzelnen Strahlenbündel zwar etwas auf, parallelisiert die Strahlenbündel zueinander jedoch erheblich. Je paralleler die Strahlenbündel innerhalb des distalen Umlenkprismas 22 verlaufen, desto kompakter kann dieses gebaut werden. Dadurch kann eine höhere numerische Apertur durch das Umlenkprisma 22 geleitet werden. Eine höhere numerischer Apertur, wie sie für Bildsensoren mit einer kleinen Pixelfrequenz benötigt werden, führt ihrerseits zu einem größeren Strahlbündeldurchmesser. Ein größerer Strahlbündeldurchmesser verursacht bei Reflektionen an geneigten Flächen eine relativ große Ellipse. Je flacher die Reflektionsfläche steht, desto größer ist der Reflektionsbereich.

Das distale Umlenkprisma 22 ist aus drei Abschnitten 22a, 22b und 22c aufgebaut.

Die einfallenden Strahlen 64 treffen auf die erste Reflektionsfläche 60 und werden von dieser in Richtung der zweiten Reflektionsfläche 62 reflektiert. Dort treffen die Strahlen unter einem Winkel größer des Grenzwinkels der inneren Totalreflektion auf. Die zweite Reflektionsfläche 62 totalreflektiert den Hauptstrahl 64 des Mittenbündels so, dass dieser colinear mit der optischen Achse 34 des nachfolgenden optischen Linsensystemes verläuft.

Die Eintrittsfläche 59 ist stets senkrecht zur Blickrichtung 57 ausgerichtet. Die Neigung der ersten Reflektionsfläche 60 bestimmt sich über den Winkel der auftreffenden Strahlen nach der Reflektion an der zweiten Reflektionsfläche 62 und der jeweiligen Blickrichtung. Die Neigung der zweiten Reflektionsfläche 62 wird aus den Brechzahlverhältnissen der drei Prismenabschnitte 22a, 22b und 22c und dem Strahlenbündeldurchmesser bestimmt.

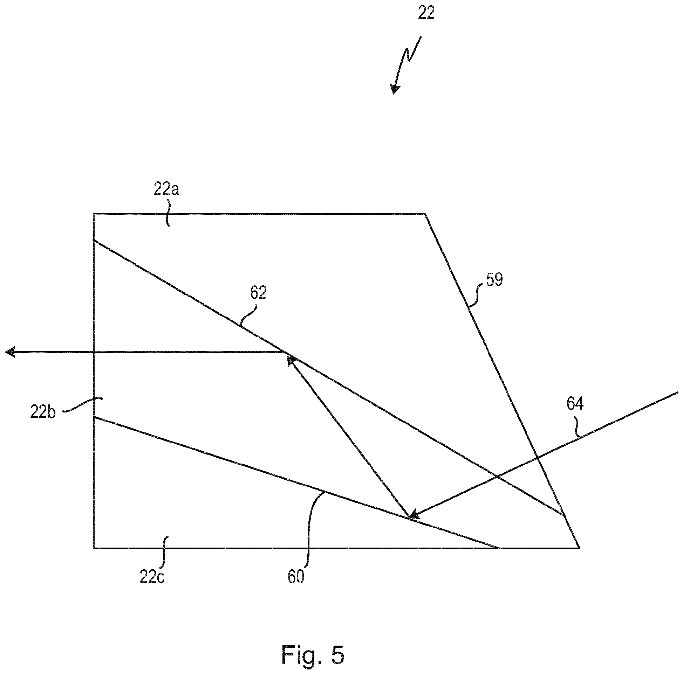

Aus der Darstellung von Fig. 5 ist ersichtlich, dass lediglich die Abschnitte 22a und 22b optisch aktiv sind. Dem Abschnitt 22c kommt nur eine mechanische Bedeutung zu. Das heißt, die gesamte Anordnung würde auch ohne den Abschnitt 22c funktionieren, dieser ist allerdings vorteilhaft für die Gesamtstabilität des Prismas. Zusätzlich bietet der Abschnitt 22c einen Schutz für die Rückfläche und die Kanten des Teilabschnittes 22b.

Im distalen Umlenkprisma 22 wird dafür gesorgt, dass eine Aufweitung der Strahlenbündel erfolgt, die aber maximal 30 % betragen sollte.

Im optischen Linsensystem 24 ist die Strahlenführung so, dass möglichst keine Reflektionen am umfänglichen Rand erfolgen, so dass dadurch kein Falschlicht entsteht, das auf dem Bildsensor 32 zu Unschärfen führen könnte. Die vom optischen Linsensystem 24 austretenden Strahlenbündel werden an einer unter 45° zur optischen Achse 34 stehenden Reflektionsfläche 33 des proximalen zweiten Umlenkprismas 30 um 90° umgelenkt und auf die Bildeintrittsebene 50 des Bildsensors 32 geleitet.

Die Anordnung des distalen ersten Umlenkprismas 22 mit seinem radial seitlich überstehenden Abschnitt 44 und dem anschließenden Linsensystem 24 erlaubt ein hochaufgelöstes Bild zu erzeugen. Mit anderen Worten ist der Aperturwinkel, also die numerische Apertur der Strahlenbündel 66 etc., so groß, dass ein hochscharfes Bild, insbesondere mit einer Pixelgröße kleiner 3 µm auf dem Bildsensor 32 erzeugt werden kann.

## Patentansprüche

1. Beobachtungsinstrument mit einem Hohlkörper (12), der als Hohlschaft eines bestimmten Innendurchmessers (14) ausgebildet ist, wobei in einem Endbereich (16) des Hohlkörpers (12) ein opto-elektronisches Bildaufnehmersystem (18) angeordnet ist, das bildeintrittseitig ein optisches Linsensystem (24) mit einem zylinderförmigen Abschnitt (26) und anschließend einen Bildsensor (32) aufweist, der das vom optischen Linsensystem (24) kommende Bild in elektrische Bildsignale wandelt, wobei sich die optische Achse (34) des Linsensystems (24) in Richtung der Längsachse des Hohlkörpers (12) erstreckt, wobei der Außendurchmesser (28) des zylinderförmigen Abschnitts (26) des Linsensystems (24) geringer ist als der Innendurchmesser (14) des Hohlkörpers (12), und zwar derart, dass zwischen einer Innenseite des Hohlkörpers (12) und der Außenseite (36) des optischen Linsensystems (24) ein ausreichender Zwischenraum (38) verbleibt, durch den weitere Bauelemente im Hohlkörper (12) durchführbar sind, wobei bei einer Blickrichtung (57) von größer 0°bis kleiner 90°aus der optischen Achse (34) des Bildsystems heraus bildeintrittsseitig am Linsensystem (24) ein distales Umlenkprisma (22) angeordnet ist, wobei das distale Umlenkprisma (22) einen sich seitlich über den Außendurchmesser (28) des zylinderförmigen Abschnitts (26) des Linsensystems (24) hinauserstreckenden Abschnitt (44) aufweist, und wobei die Bildeintrittsebene (50) des Bildsensors (32) parallel zur optischen Achse (34) verläuft, wobei sich das distale Umlenkprisma (22) seitlich in derselben Richtung über den Außendurchmesser (28) des zylinderförmigen Abschnitts (26) des Linsensystems (24) hinauserstreckt, in der die Bildeintrittsebene (50) des Bildsensors (32) zum zylinderförmigen Abschnitt (26) des Linsensystems (24) seitlich nach außen versetzt ist, **dadurch gekennzeichnet, dass** zwischen dem optischen Linsensystem (24) und dem Bildsensor (32) ein proximales Umlenkprisma (30) angeordnet ist, dessen Bildaustrittsfläche (31) auf der Bildeintrittsebene (50) des Bildsensors (32) liegt, und dass das den Bildsensor (32) aufweisende opto-elektronische Bildaufnehmersystem (18) in einem distalen Endbereich (16) des als Hohlschaft ausgebildeten Hohlkörpers (12) aufgenommen ist.

2. Beobachtungsinstrument nach Anspruch 1, **dadurch gekennzeichnet, dass** die Bildeintrittsebene (50) des Bildsensors (32) gegenüber dem zylinderförmigen Abschnitt (26) des Linsensystems (24) radial nach außen versetzt ist

3. Beobachtungsinstrument nach Anspruch 2, **dadurch gekennzeichnet, dass** das distale Umlenkprisma (22) sich seitlich in derselben Richtung radial nach außen erstreckt, in der der Bildsensor (50) nach außen versetzt ist.

4. Beobachtungsinstrument nach einem der Ansprüche 1 bis 3, **dadurch gekennzeichnet, dass** das distale Umlenkprisma (22) einen Öffnungswinkel (58) von größer/gleich 50° aufweist.

5. Beobachtungsinstrument nach einem der Ansprüche 1 bis 4, **dadurch gekennzeichnet, dass** das distale Umlenkprisma (22) derart aufgebaut ist, dass innerhalb des Umlenkprismas (22) eine Strahlaufweitung kleiner/gleich 30 % erfolgt.

6. Beobachtungsinstrument nach einem der Ansprüche 1 bis 5, **dadurch gekennzeichnet, dass** vor dem distalen Umlenkprisma (22) eine negativ brechende Linse (20) angeordnet ist, die den eintretenden Strahlen (64) einen paralleleren Verlauf verleihen.

7. Beobachtungsinstrument nach einem der Ansprüche 1 bis 6, **dadurch gekennzeichnet, dass** der Innenraum des Hohlkörpers (12) zumindest im Endbereich (16) des Hohlkörpers (12), in dem das opto-elektronische Bildaufnehmersystem (18) angeordnet ist, durch zumindest einen Trennsteg (70) derart aufgeteilt ist, dass in einem dadurch entstehenden Raum (72) das opto-elektronische Bildaufnehmersystem (18) passend aufnehmbar ist.

8. Bildaufnehmersystem nach Anspruch 7, **dadurch gekennzeichnet, dass** der Trennsteg (70) der Außenkontur des seitlich überstehenden Abschnittes (44) distalen Umlenkprismas (22) angepasst ist.

9. Beobachtungsinstrument nach Anspruch 7 oder 8, **dadurch gekennzeichnet, dass** der zumindest eine Trennsteg (22) derart ausgebildet ist, dass das opto-elektronische Bildaufnehmersystem (18) zur Montage von distal nach proximal in einen durch diesen Trennsteg (22) entstehenden Raum (72) einführbar ist.

10. Beobachtungsinstrument nach einem der Ansprüche 7 bis 9, **dadurch gekennzeichnet, dass** sich der radial seitlich vorstehende Abschnitt (44) des distalen Umlenkprismas (22) bis an den Trennsteg (70) heran erstreckt.

11. Beobachtungsinstrument nach einem der Ansprüche 1 bis 10, **dadurch gekennzeichnet, dass** der radial seitlich vorstehende Abschnitt (44) des distalen Umlenkprismas (22) sich zumindest bis zu einer Ebene erstreckt, die durch die Bildeintrittsebene (50) des Bildsensors (32) aufgespannt ist.

12. Beobachtungsinstrument nach Anspruch 11, **dadurch gekennzeichnet, dass** der radial seitlich vorstehende Abschnitt (44) des distalen Umlenkprismas (22) sich über eine Ebene (52) hinaus erstreckt, die durch die Rückseite (42) des Bildsensors (32) aufgespannt ist.

## Claims

1. Observation instrument comprising a hollow body (12) that is arranged as a hollow shaft having a specific internal diameter (14), wherein in an end region (16) of the hollow body (12) an optoelectronic image recording system (18) is arranged that comprises on the image entrance side an optical lens system (24) having a cylindrical portion (26) and subsequently an image sensor (32) that converts the image transmitted by the optical lens system (24) into electrical image signals, wherein the optical axis (34) of the lens system (24) extends in the direction of the longitudinal axis of the hollow body (12), wherein the external diameter (28) of the cylindrical portion (26) of the lens system (24) is less than the internal diameter (14) of the hollow body (12), namely such that between an inner side of the hollow body (12) and the outer side (36) of the optical lens system (24) a sufficient interspace (38) remains, through which further components can be led in the hollow body (12), wherein at a viewing direction (57) of greater than 0° to less than 90° from the optical axis (34) of the image system a distal deflection prism (22) is arranged on the image entrance side at the lens system (24), wherein the distal deflection prism (22) comprises a portion (44) extending laterally beyond the external diameter (28) of the cylindrical portion (26) of the lens system (24), and wherein the image entrance plane (50) of the image sensor (32) runs approximately parallel to the optical axis (34), wherein the distal deflection prism (22) extends laterally beyond the outer diameter (28) of the cylindrical portion (26) of the optical lens system (24) in the same direction in which the image entrance plane (50) of the image sensor (32) is offset laterally outwards to the cylindrical portion (26) of the lens system (24), **characterized in that** between the optical lens system (24) and the image sensor (32) a proximal deflection prism (30) is arranged whose image exit surface (31) lies on the image entrance plane (50) of the image sensor (32), and that the optoelectronic image recording system (18) that is provided with the image sensor (32) is arranged in a distal end region (16) of the hollow body (12) that is arranged as a hollow shaft.

2. Observation instrument according to Claim 1, **characterized in that** the image entrance plane (50) of the image sensor (32) is offset radially outwards relative to the cylindrical portion (26) of the lens system (24).

3. Observation instrument according to Claim 2, **characterized in that** the distal deflection prism (22) extends radially outwards laterally in the same direction in which the image sensor (50) is offset outwards.

4. Observation instrument according to any of Claims 1 to 3, **characterized in that** the distal deflection prism (22) comprises an aperture angle (58) of greater than/equal to 50°.

5. Observation instrument according to any of Claims 1 to 4, **characterized in that** the distal deflection prism (22) is constructed in such a way that beam expansion of less than/equal to 30% is effected within the deflection prism (22).

6. Observation instrument according to any of Claims 1 to 5, **characterized in** in front of the distal deflection prism (22) that a negative refractive index lens (20) is arranged that imparts a more parallel course to the entering rays (64).

7. Observation instrument according to any of Claims 1 to 6, **characterized in that** the interior of the hollow body (12), at least in the end region (16) of the hollow body (12) in which the optoelectronic image recording system (18) is arranged, is divided by at least one separating web (70) in such a way that the optoelectronic image recording system (18) can be accommodated fittingly in a space (72) formed in this way.

8. Observation instrument according to Claim 7, **characterized in that** the separating web (70) is adapted to the outer contour of the laterally projecting portion (44) of the distal deflection prism (22).

9. Observation instrument according to Claim 7 or 8, **characterized in that** the at least one separating web (22) is formed in such a way that the optoelectronic image recording system (18) can be introduced for the purpose of mounting from distal to proximal into a space (72) formed as a result of said separating web (22).

10. Observation instrument according to any of Claims 7 to 9, **characterized in that** the radially laterally projecting portion (44) of the distal deflection prism (22) extends right up to the separating web (70).

11. Observation instrument according to any of Claims 1 to 10, **characterized in that** the radially laterally projecting portion (44) of the distal deflection prism (22) extends at least up to a plane which is spanned by the image entrance plane (50) of the image sensor (32).

12. Observation instrument according to Claim 11, **characterized in that** the radially laterally projecting portion (44) of the distal deflection prism (22) extends beyond a plane (52) spanned by the rear side (42) of the image sensor (32).

## Revendications

1. Instrument d'observation comportant un corps creux (12) qui est réalisé sous la forme d'un arbre creux ayant un diamètre intérieur déterminé (14), dans lequel un système d'acquisition d'images optoélectronique (18) est disposé dans une région d'extrémité (16) du corps creux (12), lequel système comporte un système de lentilles optique (24) ayant une partie cylindrique (26) du côté d'entrée d'image, puis un capteur d'image (32) convertissant l'image provenant du système de lentilles optique (24) en des signaux d'images électriques, dans lequel l'axe optique (34) du système de lentilles (24) s'étend dans la direction de l'axe longitudinal du corps creux (12), dans lequel le diamètre extérieur (28) de la partie cylindrique (26) du système de lentilles (24) est inférieur au diamètre intérieur (14) du corps creux (12), de telle sorte qu'un espace intermédiaire (38) suffisant subsiste entre une face intérieure du corps creux (12) et la face extérieure (36) du système de lentilles optique (24), à travers lequel d'autres composants peuvent être introduits dans le corps creux (12), dans lequel un prisme de déflexion distal (22) est disposé du côté d'entrée d'image du système de lentilles (24) pour une direction de visée (57) comprise entre plus de 0° et moins de 90° par rapport à l'axe optique (34) du système d'image, dans lequel le prisme de déflexion distal (22) comporte une partie (44) s'étendant latéralement au-delà du diamètre extérieur (28) de la partie cylindrique (26) du système de lentilles (24), et dans lequel le plan d'entrée d'image (50) du capteur d'images (32) s'étend parallèlement à l'axe optique (34), dans lequel le prisme de déflexion distal (22) s'étend latéralement au-delà du diamètre extérieur (28) de la partie cylindrique (26) du système de lentilles (24) dans la même direction que celle dans laquelle le plan d'entrée d'image (50) du capteur d'images (32) est décalé latéralement vers l'extérieur jusqu'à la partie cylindrique (26) du système de lentilles (24),
**caractérisé en ce qu'**un prisme de déflexion proximal (30) est disposé entre le système de lentilles optique (24) et le capteur d'image (32), dont la surface de sortie d'image (31) est située sur le plan d'entrée d'image (50) du capteur d'image (32), et **en ce que** le système d'acquisition d'images optoélectronique (18) comportant le capteur d'images (32) est reçu dans une région d'extrémité distale (16) du corps creux (12) réalisé sous la forme d'un arbre creux.

2. Instrument d'observation selon la revendication 1, **caractérisé en ce que** le plan d'entrée d'image (50) du capteur d'image (32) est décalé radialement vers l'extérieur par rapport à la partie cylindrique (26) du système de lentilles (24).

3. Instrument d'observation selon la revendication 2, **caractérisé en ce que** le prisme de déflexion distal (22) s'étend radialement vers l'extérieur et latéralement dans la même direction que celle dans laquelle le capteur d'image (50) est décalé vers l'extérieur.

4. Instrument d'observation selon l'une quelconque des revendications 1 à 3, **caractérisé en ce que** le prisme de déflexion distal (22) présente un angle d'ouverture (58) supérieur ou égal à 50°.

5. Instrument d'observation selon l'une quelconque des revendications 1 à 4, **caractérisé en ce que** le prisme de déflexion distal (22) est construit de manière à ce qu'un élargissement du faisceau inférieur ou égal à 30 % ait lieu à l'intérieur du prisme de déflexion (22).

6. Instrument d'observation selon l'une quelconque des revendications 1 à 5, **caractérisé en ce qu'**une lentille réfractive négative (20) est disposée en amont du prisme de déflexion distal (22), et confère aux faisceaux d'entrée (64) une trajectoire plus parallèle.

7. Instrument d'observation selon l'une des revendications 1 à 6, **caractérisé en ce que** l'intérieur du corps creux (12) est divisé par au moins une bande de séparation (70), au moins dans la zone d'extrémité (16) du corps creux (12) dans laquelle est disposé le système d'acquisition d'images optoélectronique (18), de manière à ce que le système d'acquisition d'images optoélectronique (18) puisse être logé de manière appropriée dans un espace (72) ainsi créé.

8. Système d'acquisition d'images selon la revendication 7, **caractérisé en ce que** la bande de séparation (70) est adaptée au contour extérieur du prisme de déflexion distal (22) de la partie dépassant latéralement (44).

9. Instrument d'observation selon la revendication 7 ou 8, **caractérisé en ce que** ladite au moins une bande de séparation (22) est réalisée de manière à ce que le système d'acquisition d'images optoélectronique (18) puisse être introduit dans un espace (72) créé par ladite bande de séparation (22) pour un montage distal à proximal.

10. Instrument d'observation selon l'une quelconque des revendications 7 à 9, **caractérisé en ce que** la partie dépassant latéralement et radialement (44) du prisme de déflexion distal (22) s'étend jusqu'à la bande de séparation (70).

11. Instrument d'observation selon l'une quelconque des revendications 1 à 10, **caractérisé en ce que** la partie dépassant latéralement et radialement (44) du prisme de déflexion distal (22) s'étend au moins jusqu'à un plan passant par le plan d'entrée d'image (50) du capteur d'image (32).

12. Instrument d'observation selon la revendication 11, **caractérisé en ce que** la partie dépassant latéralement et radialement (44) du prisme de déflexion distal (22) s'étend au-delà d'un plan (52) passant par la face arrière (42) du capteur d'images (32).
